(19) Europäisches Patentamt
European Patent Office
Office européen des brevets

(11) Veröffentlichungsnummer : **0 046 495**
**B1**

# (12) EUROPÄISCHE PATENTSCHRIFT

(45) Veröffentlichungstag der Patentschrift : **23.05.84**

(51) Int. Cl.³ : **C 07 C107/00**// C09B29/085, C07D475/00

(21) Anmeldenummer : **81105333.9**

(22) Anmeldetag : **09.07.81**

## (54) Verfahren zur Herstellung von N-(D)-Ribityl-2-phenylazo-4,5-dimethylanilin.

(30) Priorität : **08.08.80 DE 3030054**

(43) Veröffentlichungstag der Anmeldung : **03.03.82 Patentblatt 82/09**

(45) Bekanntmachung des Hinweises auf die Patenterteilung : **23.05.84 Patentblatt 84/21**

(84) Benannte Vertragsstaaten : **BE CH DE FR GB IT LI NL**

(56) Entgegenhaltungen :
**AT-B- 168 083**
**FR-A- 1 579 589**
**JP-A-39 006 665**
**US-A- 2 477 560**
**W.H. SEBRELL, R.S. HARRIS: "The vitamins; chemistry, physiology, pathology, methods", 2. Ausgabe, Band V, 1972, Academic Press, Seiten 19-26 New York, U.S.A.**
**Helv. Chim. Acta, Band 19, 1936, S. 264-269**

(73) Patentinhaber : **BASF Aktiengesellschaft**
**Carl-Bosch-Strasse 38**
**D-6700 Ludwigshafen (DE)**

(72) Erfinder : **Schmidt, Wolfram, Dr.**
**Im Rosengarten Ost 8**
**D-6701 Friedelsheim (DE)**
Erfinder : **Paust, Joachim, Dr.**
**Ringstrasse 3**
**D-6701 Neuhofen (DE)**
Erfinder : **Nuerrenbach, Axel, Dr.**
**Koenigsberger Strasse 7**
**D-6718 Gruenstadt (DE)**

**EP 0 046 495 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein verbessertes Verfahren zur Herstellung von N-(D)-Ribityl-2-phenylazo-4,5-dimethylanilin (I)

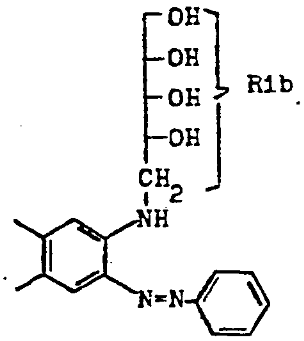

(I)

einem wichtigen Vorprodukt für die Herstellung von Vitamin $B_2$ (Riboflavin, II)

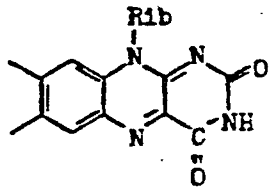

(II)

Aus der Monographie « The Vitamins ; Chemistry, Physiology, Pathology, Methods » von W.H. Sebrell und R.S. Harris, Academic Press, 2. Auflage, Band V (1972), Seite 22 ist es bekannt, 2,3-Dimethylanilin mit (D)-Ribose und Wasserstoff zum N-Ribityl-2,3-dimethylanilin umzusetzen und letztere mit einem p-Alkylphenyldiazoniumchlorid zu einem N-Ribityl-2-(p-alkyl)-phenylazo-4,5-dimethylanilin zu kuppeln. Sowohl nach dieser Literaturstelle als auch nach der Originalarbeit (Helv. Chim. Acta Band 19, 1936, Seiten 264-269) geht man hierbei von reiner (D)-Ribose aus, jedenfalls findet die Verwendung von roher (D)-Ribose keine Erwähnung.

Das prinzipiell gleiche Verfahren ist aus der JP-A 396665 bekannt, demzufolge reine (D)-Ribose (III) und 3,4-Dimethylnitrobenzol (IVa) oder 3,4-Dimethylanilin (IVb) mit Wasserstoff in Gegenwart von Palladium oder Raney-Nickel zum 3,4-Dimethyl-N-(D)-ribitylanilin (V) umgesetzt wird, welches sodann wie üblich mit Phenyldiazoniumchlorid (VI) in (I) überführt werden kann.

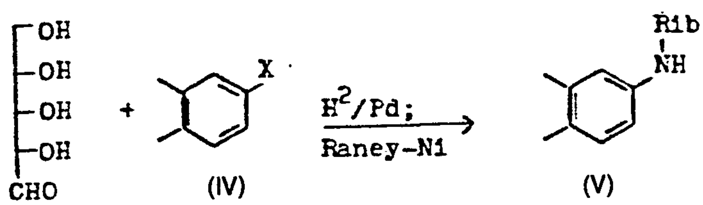


(III) (IV) (V)

IVa: $X=NO_2$

IVb: $X=NH_2$

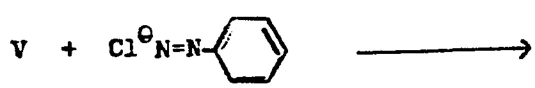

(I)

(VI)

Die technische Anwendung dieses Verfahrens ist jedoch mit erheblichen Problemen verbunden, da reine (D)-Ribose einerseits durch Epimerisierung von Arabinose mit wirtschaftlich vertretbarem Aufwand bisher nicht zugänglich ist, und andererseits (V) aus den mit technischer Ribose (« Rohribose », Riboseanteil etwa 50 bis 70 %) erhältlichen Reaktionsgemischen nur schwierig abgetrennt werden kann. Da die in der Rohribose enthaltenen isomeren Zucker, hauptsächlich die D-Arabinose (VII), gleichzeitig

mit IV reagieren, muß man darüber hinaus entsprechende Verluste an den wertvollen Ausgangsstoffen (IV) in Kauf nehmen.

Nach dem Verfahren der US-A 2 477 560, welches die Herstellung von V aus III und IVb auf prinzipiell die gleiche Weise betrifft, kann man zwar auch von Rohribose ausgehen, jedoch wird hier unter Rohribose ein Produkt verstanden, welches durch Reduktion von Ribonolacton mit Natriumamalgam erhältlich ist. Eine derartige Herstellung von Rohribose ist aber wegen der verfahrenstechnischen Schwierigkeiten mit Amalgam als Reaktionspartner technisch überholt.

Verluste an IV treten auch bei dem Verfahren der AT-PS 168 083 auf, nach welchem man eine durch Amalgamreduktion von Ribonolacton erhaltene Rohribose mit etwa 50 % Arabinose-Anteil mit IVb und Borsäure in die Borsäureester der Schiffschen Base aus IVb und III überführt.

Aus dem so erhaltenen Reaktionsgemisch läßt sich dieser Borsäureester durch Kristallisation in reiner Form isolieren. Das Kristallisat wird abgetrennt, getrocknet, wieder in Wasser oder in Wasser/Alkohol gelöst und zu V hydriert. Sodann wird V durch Kristallisation gewonnen und in wäßriger Salzsäure gelöst. Die Lösung wird mit Aktivkohle gereinigt, wonach V abermals kristallisiert wird. Anschließend wird eine wäßrige Lösung des reinen V der Azokupplung mit VI unterworfen, wonach man das Verfahrensprodukt I durch Kristallisation isoliert.

Wenngleich man I auf diese Weise in reiner Form gewinnen kann, so ist dieses Verfahren wegen der Verluste an IVb und der zahlreichen Einzeloperationen technisch nicht brauchbar.

Aus der FR-A 1 579 589 ist es schließlich bekannt, V zur Herstellung von I in Form einer wäßrigen Suspension mit einer Lösung von VI umzusetzen.

Ferner ist es aus der CS-PS 149 472 bekannt, (D)-Arabinose mittels Molybdänsäure zu epimerisieren, wobei man Roh-(D)-Ribosen mit etwa 70 % (D)-Riboseanteil erhält.

Der Erfindung lag die Aufgabe zugrunde, I ausgehend von Rohribose auf technisch einfachere und wirtschaftlichere Weise herzustellen.

Demgemäß wurde ein verbessertes Verfahren zur Herstellung von N-(D)-Ribityl-2-phenylazo-4,5-dimethylanilin (I) durch Umsetzung von roher (D)-Rohribose, also technisch erhältlicher Gemische aus (D)-Ribose (III) und anderen Zuckern, mit 3,4-Dimethylanilin (IVb) und Borsäure bei 0-50 °C in wäßriger oder wäßrig-organischer Lösung oder in der Lösung eines wasserlöslichen organischen Lösungsmittels, Abtrennung des hiernach auskristallisierten Borsäureesters der Schiffschen Base aus III und IVb, Hydrierung dieses Borsäureesters in wäßriger oder wäßrig-organischer Lösung bei 20-70 °C und unter einem Wasserstoffdruck von 10-100 bar mit Wasserstoff in Gegenwart eines Hydrierkatalysators, Umsetzung der vom Hydrierkatalysator befreiten Lösung mit einer sauren Phenyldiazoniumsalzlösung und Isolierung des hierbei entstehenden Verfahrensproduktes I durch Kristallisation gefunden, welches dadurch gekennzeichnet ist, daß man hierzu eine solche rohe (D)-Rohribose verwendet, die durch Epimerisierung von (D)-Arabinose mittels Molybdänsäure als Epimerisierungskatalysator hergestellt worden ist, und daß man IVb in etwa äquimolaren Mengen, bezogen auf den Gehalt an (D)-Ribose in der rohen (D)-Rohribose, einsetzt.

Besonders bemerkenswert an diesem Verfahren ist, daß man lediglich etwa äquimolare Mengen, bezogen auf den Ribosegehalt, des teuren 3,4-Dimethylanilins (IVb), benötigt, wodurch sich die Verwendung der Rohribose überhaupt erst wirtschaftlich gestaltet. Außerdem kann sich die Azokupplung ohne Isolierung der Zwischenstufe (V) unmittelbar an die Hydrierung des Borsäureesters der Schiffschen Base aus III und IVb anschließen.

Diese Vorteile bedingen eine wesentliche Vereinfachung der Synthese von I und damit des Vitamin $B_2$ (II).

Die erfindungsgemäß einzusetzende Rohribose enthält etwa 70 % (D)-Ribose und ist durch Epimerisierung von (D)-Arabinose nach Bilek (CS-PS 149 472) mittels Molybdänsäure zugänglich.

Die molare Menge des 3,4-Dimethylanilins (IVb) entspricht, will man die Ribose voll ausnutzen, dem Ribosegehalt in der Rohribose.

Die Borsäure verwendet man zweckmäßigerweise in Mengen von 0,7 bis 1 mol pro Mol der Ribose. Man kann die Ribose zunächst mit der Borsäure verestern und danach mit IV umsetzen, jedoch empfiehlt es sich im allgemeinen, Borsäure und IVb gleichzeitig auf die Ribose einwirken zu lassen.

Der Borsäureester der Schiffschen Base ist im wäßrigen oder wäßrig-organischen Milieu schwer löslich, weshalb er fast vollständig — gegebenenfalls nach Abkühlung des Reaktionsgemisches — zur Kristallisation gebracht werden kann. Als Lösungsmittel empfehlen sich Wasser, Methanol, Ethanol, Wasser-Methanol- und Wasser-Ethanol-Gemische.

Vorzugsweise führt man die Umsetzung bei Temperaturen von 30-50 °C aus. Die Reaktionszeiten betragen bei diesen Temperaturen etwa 0,2-1 Stunde.

Nach beendeter Reaktion läßt man den Borsäureester zweckmäßigerweise zunächst durch allmähliche Temperatursenkung teilweise und danach bei tieferen Temperaturen — etwa 0-20 °C — vollständig auskristallisieren. Man trennt das Kristallisat ab und verwendet es zweckmäßigerweise in feuchter Form weiter. Eine Wäsche des Kristallisats empfiehlt sich allenfalls zur Entfernung von überschüssigem IVb. Hierzu kann z. B. Methylenchlorid oder Ether verwendet werden.

Zur Hydrierung des Borsäureesters der Schiffschen Base löst man diesen zweckmäßigerweise in der 5- bis 10-fachen Menge Wasser oder eines wäßrig-organischen Lösungsmittels, z. B. Methanol/Wasser oder Ethanol/Wasser. Im übrigen wird die Hydrierung wie üblich vorgenommen, jedoch sollte eine

Temperatur von 70 °C nicht überschritten werden.

Nach Abtrennung des Hydrierkontakts wird die so erhaltene Lösung des Borsäureesters von V ebenfalls wie üblich unmittelbar oder nach Abtrennung des organischen Lösungsmittels bei (– 5)– 5 °C mit einer Phenyldiazoniumsalzlösung umgesetzt. Unter den Bedingungen dieser Umsetzung wird die Borsäure wieder abgespalten.

Die Aufarbeitung des Reaktionsgemisches auf I erfolgt ebenfalls in an sich bekannter Weise.

Die Ausbeuten, bezogen auf den Riboseanteil in der Rohribose, liegen etwa zwischen 75 und 80 %.

## Beispiel

Als Rohribose wurde eine nach Bilek epimerisierte (D)-Arabinose verwendet, die wie folgt hergestellt wurde :

100 g (D)-Arabinose wurden in 500 ml Wasser zusammen mit 1 g Molybdänsäure 3 h auf 90-100 °C erhitzt. Nach dem Eindampfen der wäßrigen Lösung wurde das zurückbleibende Gemisch bei 65 °C in 230 ml einer Mischung aus 90 Vol.% Ethanol und 10 Vol.% Wasser gelöst, wonach die Lösung abgekühlt wurde. Hierbei kristallisierten 70 g Arabinose aus. Die Mutterlauge enthielt eine 70 %ige Rohribose, also 21 g (140 mmol Ribose (III) und 9 g sonstiger Zucker.

Diese Lösung wurde sodann auf etwa 100 ml eingeengt und bei 45 °C mit 16,9 g (140 mmol) 3,4-Dimethylanilin (IVb) und 12,4 g (200 mmol) Borsäure versetzt. Nachdem eine klare Lösung entstanden war, wurde diese auf 20 °C abgekühlt, wobei der Borsäureester der Schiffschen Base aus III und IVb auszukristallisieren begann. Nach 2 Stunden wurde die Kristallisation in 2 weiteren Stunden bei 0 °C zu Ende geführt. Das Kristallisat wurde abfiltriert und mit Ether gewaschen. Die Ausbeute an dem Borsäureester betrug 90 %, bezogen auf III.

Das noch feuchte Kristallisat wurde sodann in einer Mischung aus 270 ml Methanol und 15 ml Wasser gelöst und in Gegenwart von 11 g Raney-Nickel bei 55 °C und 30 bar Wasserstoffdruck hydriert. Die Ausbeute an 3,4-Dimethyl-N-ribitylanilin betrug 95 % (entspricht 30,5 g = 120 mmol).

Die bei dem Hydrierungsschritt erhaltene Lösung wurde auf etwa 50 ml eingeengt und danach mit 100 ml konzentrierter Salzsäure und 40 ml Essigsäure versetzt.

Hierzu wurde bei (– 5 °C) eine Phenyldiazoniumchloridlösung gegeben, die auf übliche Weise aus 12,4 g (133 mmol) Anilin, 9,1 g $NaNO_2$ in 90 ml 15 gew.%iger Salzsäure hergestellt worden war. Der pH-Wert betrug hierbei zwischen 1 und 1,5. Nach beendeter Kupplung wurde der pH-Wert mit NaOH auf 3,5 gestellt, wonach das Verfahrensprodukt I auskristallisierte. Das Kristallisat wurde abgetrennt und mit Wasser gewaschen. Die Ausbeute an I betrug bei der Kupplung 90 %, und bezogen auf die eingesetzte Ribose betrug sie 77 %.

**Anspruch**

Verfahren zur Herstellung von N-(D)-Ribityl-2-phenylazo-4,5-dimethylanilin (I) durch Umsetzung von roher (D)-Rohribose, also technisch erhältlicher Gemische aus (D)-Ribose (III) und anderen Zuckern, mit 3,4-Dimethylanilin (IVb) und Borsäure bei 0-50 °C in wäßriger oder wäßrig-organischer Lösung oder in der Lösung eines wasserlöslichen organischen Lösungsmittels, Abtrennung des hiernach auskristallisierten Borsäureesters der Schiffschen Base aus III und IVb, Hydrierung dieses Borsäureesters in wäßriger oder wäßrig-organischer Lösung bei 20-70 °C und unter einem Wasserstoffdruck von 10-100 bar mit Wasserstoff in Gegenwart eines Hydrierkatalysators, Umsetzung der vom Hydrierkatalysator befreiten Lösung mit einer sauren Phenyldiazoniumsalzlösung und Isolierung des hierbei entstehenden Verfahrensproduktes I durch Kristallisation, dadurch gekennzeichnet, daß man hierzu eine solche rohe (D)-Rohribose verwendet, die durch Epimerisierung von (D)-Arabinose mittels Molybdänsäure als Epimerisierungskatalysator hergestellt worden ist, und daß man IVb in etwa äquimolaren Mengen, bezogen auf den Gehalt an (D)-Ribose in der rohen (D)-Rohribose, einsetzt.

**Claim**

A process for the preparation of N-(D)-ribityl-2-phenylazo-4,5-dimethylaniline (I) by reacting crude (D)-ribose, i. e. an industrial mixture of (D)-ribose (III) and other sugars, with 3,4-dimethylaniline (IVb) and boric acid at 0 to 50 °C in aqueous or aqueous-organic solution or in solution in a water-soluble organic solvent, allowing the boric acid ester of the Schiff base obtained from III and IVb to crystallize out and then separating it off, hydrogenating this boric acid ester in aqueous or aqueous-organic solution, at 20 to 70 °C and under a hydrogen pressure of from 10 to 100 bar, with hydrogen in the presence of a hydrogenation catalyst, freeing the solution from catalyst and then reacting it with an acid phenyldiazonium salt solution, and isolating the resulting product I by crystallization, wherein there is used a crude (D)-ribose which has been obtained by epimerizing (D)-arabinose with molybdic acid as epimerization catalyst, and wherein IVb is employed in an approximately molar amount, based on the content of (D)-ribose in the crude (D)-ribose.

**Revendication**

Procédé pour la préparation de N-(D)-ribityl-2-phénylazo-4,5-diméthylaniline (I) par réaction de (D)-ribose brut, c'est-à-dire de mélanges obtenus industriellement de (D)-ribose (III) et d'autres sucres, avec la 3,4-diméthylaniline (IVb) et l'acide borique, à 0-50 °C, en solution aqueuse ou aqueuse-organique ou dans la solution d'un solvant organique soluble dans l'eau, séparation de l'ester borique de la base de Schiff dérivée de III et IVb qui se sépare alors à l'état cristallin, hydrogénation de cet ester borique en solution aqueuse ou aqueuse-organique, à 20-70 °C et sous une pression d'hydrogène de 10 à 100 bars, avec de l'hydrogène et présence d'un catalyseur d'hydrogénation, réaction de la solution débarrassée du catalyseur d'hydrogénation avec une solution acide de sel de phényldiazonium et isolement du produit final qui est ainsi formé, caractérisé en ce qu'on utilise à cet effet un (D)-ribose brut qui a été préparé par épimérisation de (D)-arabinose au moyen d'acide molybdique servant de catalyseur d'épimérisation, et en ce qu'on met à réagir IVb dans des proportions à peu près équimolaires, par rapport à la teneur en (D)-ribose du (D)-ribose brut.